# EUROPEAN PATENT APPLICATION

(11) **EP 4 725 410 A1**
(43) Date of publication of application: **15.04.2026**
(21) Application number: 24205333.8
(22) Date of filing: 08.10.2024
(51) Int. Cl.: A61B 5/103, A61B 5/11, A61B 5/00

(54) **SYSTEM AND METHOD FOR PREDICTING FALL RISK IN ELDERLY PATIENTS USING VIDEO GAME-BASED ASSESSMENT**

(71) Applicant: Otto-von-Guericke-Universität Magdeburg, 39106 Magdeburg (DE)
(72) Inventor: MERTENS, Peter, 39104 Magdeburg (DE); MING, Antao, 39120 Magdeburg (DE)

(57) **Abstract**

The present disclosure provides a video game-based fall risk assessment system (100) designed for individuals (120). The system comprises sensor-equipped insoles (102) embedded in gaming slippers, featuring pressure sensors to monitor plantar foot pressure at key locations. An electronic control unit (104) processes this data and a video gaming application on a smart device (106) uses real-time pressure data to facilitate gameplay, evaluating skills relevant to fall prevention, such as balance and reaction time. A server (108) receives gameplay data, extract specific performance parameters (including reaction time and success rate) using a machine learning model (116), and analyze these parameters to assess the individual's balance and stability. The machine learning model (116) evaluates fall risk based on seated or a combination of seated/standing data, employing statistical analysis techniques. Finally, the system classifies individuals into "no/low risk of falls" or "high risk of falls" categories, considering posture-specific risks for accurate fall risk assessment.

## Description

### TECHNICAL FIELD OF DISCLOSURE

The present disclosure generally relates to fall risk prediction and prevention. More particularly, the present disclosure relates to a system and method utilizing sensor-equipped insoles and video game-based assessment tools to evaluate and predict fall risk in individuals, particularly elderly patients with conditions such as diabetes.

### BACKGROUND

Falls pose a considerable risk to public health, particularly among the elderly population, where they are a leading cause of morbidity and mortality. Recent statistics indicate that falls are the second leading global cause of mortality resulting from accidental and unintentional injuries. Among individuals aged 65 years and older, approximately 25% experience at least one fall annually, significantly impacting their quality of life and leading to serious complications, including fractures and long-term disability. The risk of falling is further exacerbated in older adults with chronic diseases, especially those that impair joint integrity, muscle strength, balance, and cognitive function.

Polyneuropathy and cognitive decline are common conditions among elderly patients, particularly those with diabetes. Patients suffering from diabetes encounter unique challenges that increase their fall risk, including hypoglycemia, loss of protective sensation due to peripheral neuropathy, visual impairments, and fluctuating glucose levels resulting from insulin and antidiabetic therapy. These factors contribute to frailty and significantly elevate the likelihood of falls, necessitating improved fall risk assessment and prevention strategies tailored to this vulnerable population.

Over the past few decades, various assessment tools have been developed to predict fall risk in elderly individuals. Notable examples include the Hendrich II Fall Risk Model (HFRM-II), Comprehensive Geriatric Assessment (CGA), Timed "Up and Go" (TUG) test, Dynamic Gait Index (DGI), and Berg Balance Scale (BBS). However, these traditional fall risk assessment tools often exhibit low predictive values, particularly in detecting subtle changes in an individual's fall risk. Furthermore, they frequently fail to adequately account for the complex interplay of factors affecting patients with diabetes, which includes but is not limited to cognitive decline and peripheral neuropathy.

The limitations of existing fall risk assessment tools highlight a pressing need for innovative solutions that integrate advanced methodologies to improve predictive accuracy. Machine learning algorithms, specifically, have the potential to analyze complex datasets, including gameplay performance parameters, to derive insights into an individual's balance, postural stability, and motor control performance. Such an approach could enhance the accuracy of fall risk assessments and allow for more personalized interventions, ultimately reducing the incidence of falls among elderly individuals, particularly those with diabetes and other chronic conditions.

Therefore, there is a need to address the aforementioned technical drawbacks in existing technologies for fall risk assessment, particularly for elderly individuals with chronic conditions such as diabetes and cognitive decline.

### SUMMARY

The present disclosure seeks to provide an innovative solution for fall risk assessment using advanced machine learning algorithms and gameplay performance data. This invention aims to enhance predictive accuracy, particularly for elderly individuals with chronic conditions, by analyzing specific game parameters to classify individuals as "no/low risk of falls" or "high risk of falls". By employing predefined hypotheses, statistical analysis techniques, and feature selection processes, the system addresses the limitations of traditional assessment tools and improves fall risk classification, ultimately aiming to reduce fall incidence and enhance the quality of life for vulnerable populations. The object of the present disclosure is achieved by the solutions provided in the enclosed independent claims. Advantageous implementations of the present disclosure are further defined in the dependent claims.

In one aspect, a video game-based fall risk assessment system for assessing fall risk in an elderly individual is provided. The system includes a pair of sensor-equipped insoles embedded within a pair of size-matched gaming slippers. The insoles comprise eight pressure sensors positioned at defined locations within the foot, including the heel, lateral arch, metatarsals 1, 3, and 5, hallux, and toes. The system includes an electronic control unit configured to receive and process plantar foot pressure data from the pressure sensors, detect foot side automatically, and exchange data with external applications. The system includes a video gaming application installed on a smart device configured to receive the plantar foot pressure data from the electronic control unit in real-time. The gaming application comprising four video games, each video game is designed to address capabilities pertinent to prevent fall comprising skillfulness, reaction time, sensation, endurance, balance, and muscle strength. The individual interacts with the games by modulating the plantar foot pressures applied through the pair of sensor-equipped insoles to control throughout virtual tasks within the games. The system includes a server configured to (i) receive gameplay data from the smart device, (ii) extract specific game performance parameters during the gameplay, including reaction time, anticipation time, pressure application accuracy, time within target zone, time outside target zone, and success rate, using a machine learning model, (iii) analyze the extracted game performance parameters with the machine learning model to assess balance, postural stability, and motor control performance of the individual. The machine learning model is trained on both seated and standing gameplay data to evaluate fall risk, utilizing predefined hypotheses and statistical analysis techniques, which include feature selection processes and cross-validation methods, and (iv) classify the individual based on the analysis of the extracted game performance parameters into the categories "no/low risk of falls" or "high risk of falls". The classification considers posture-specific risks associated with both seated and standing positions by selecting and ranking the most predictive features for fall risk assessment. In case that the individual is not capable of performing the gaming in standing position the data set in the seated position is sufficient to classify according to the labels "no/low risk of falls" or "high risk of falls".

According to a particular embodiment, the predefined hypotheses are based on studies that correlate specific motor and cognitive skills with fall risk and the statistical analysis techniques comprise regression analysis, hypothesis testing, and correlation assessments to determine relationships between game performance parameters and fall risk.

According to a particular embodiment, the feature selection process employs algorithms to eliminate multicollinearity among the extracted game performance parameters and the cross-validation methods involve k-fold validation.

According to a particular embodiment, each sensor has a sensitivity of 3.4 mbar, ranging from 250 mbar to 7 bars, and operates with a maximum sampling rate of 500 Hz.

According to a particular embodiment, the sensor-equipped insoles are initialized to measure the plantar foot pressures at a frequency of 200 Hz and transmit the measured plantar foot pressure data to the video game application presented on the smart device in real-time.

According to a particular embodiment, the four video games comprise an apple-catching game requiring precision in maintaining target pressure for optimal positioning; a balloon-flying game controlled by forefoot pressure; a cross-pressure game requiring the application of specific pressures on designated foot areas; and an island-jumping game requiring the control of jump distances and directions using plantar pressures.

According to a particular embodiment, the game performance parameters extracted from the apple-catching game include the reaction time, the anticipation time, time inside the catching area, time outside the catching area, frequency outside the catching area, final virtual distance, and apple-caught success.

According to a particular embodiment, game performance parameters extracted from the balloon-flying game metrics include smiley count, collision frequency, minimal virtual distance to smiley, virtual deviation from ideal flying route, and normalized pressure.

According to a particular embodiment, the game performance parameters extracted from the cross-pressure game metrics include the anticipation time, time outside the optimal pressure zone, relaxation time, and normalized pressure for each foot.

According to a particular embodiment, the game performance parameters extracted from the island-jumping game metrics include attempt count, deviation from optimal pressure, the anticipation time, execution time, and mean pressure during the execution phase.

According to a particular embodiment, the machine learning model is configured to assess balance control of the individual by analyzing pressure differences between successive frames and pressure gradients between successive frames.

According to a particular embodiment, the game performance parameters are grouped into task combinations (TCs) based on game tasks, with secondary features being calculated, including sum, mean, and standard deviation of primary features across the tasks.

According to a particular embodiment, the server is configured to provide real-time feedback to the individual, including visual indicators of task success and graphical representations of applied pressure.

In another aspect, a method for assessing fall risk in elderly individuals using a video game-based fall risk assessment system is provided. The method includes the step of (i) receiving gameplay data from a smart device equipped with a video gaming application, the application includes a plurality of video games designed to enhance skills pertinent to fall prevention, including skillfulness, reaction time, sensation, endurance, balance, and muscle strength; (ii) extracting specific game performance parameters during gameplay, including reaction time, anticipation time, pressure application accuracy, time within the target zone, time outside the target zone, and success rate, utilizing a machine learning model; (iii) analyzing the extracted game performance parameters with the machine learning model to assess the balance, postural stability, and motor control performance of the individual, the machine learning model is trained on both seated and standing gameplay data to evaluate fall risk, employing predefined hypotheses and statistical analysis techniques, which include feature selection processes and cross-validation methods; and (iv) classifying the individual based on the analysis of the extracted game performance parameters into the categories "no/low risk of falls" or "high risk of falls", wherein the classification considers posture-specific risks associated with both seated and standing positions by selecting and ranking the most predictive features for fall risk assessment.

The video game-based fall risk assessment system of the present disclosure demonstrates significantly a higher accuracy of 82.8% and 88.6%, AUC-ROC 0.84 and 0.91, respectively compared to traditional tools like the Timed "Up and Go" test, Dynamic Gait Index, and Berg-Balance-Scale, which had predictive accuracies of 47.5% to 58.7%. The video game-based fall risk assessment system evaluates key capabilities such as reaction time, balance, and endurance, providing detailed, Al- driven insights into individual fall risk factors. The superior accuracy of the video game-based assessment of the present disclosure suggests that it could be integrated into routine clinical evaluations, offering a more reliable and engaging method for identifying highrisk individuals. This approach has the potential to enhance patient compliance and engagement, ultimately reducing fall-related morbidity and mortality.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF DRAWINGS

The embodiments herein will be better understood from the following detailed description with reference to the drawings, in which:
**FIG. 1** illustrates video game-based fall risk assessment system in accordance with the present disclosure.
**FIG. 2** illustrates the insole calibration process in accordance with the present disclosure.
**FIG. 3A-B** illustrates the performance of a gradient boosting machine (GBM) model used to assess the fall risk by analyzing various extracted game performance parameters in accordance with the present disclosure.
**FIG.4** illustrates a forest plot of game-based fall risk factors in accordance with the present disclosure.
**FIG. 5** illustrates gaming performance metrics as predictors of fall risk in seated and standing conditions factors in accordance with the present disclosure.
**FIG. 6A** illustrates hypothesis-driven key capabilities in seated session in accordance with the present disclosure.
**FIG. 6B** illustrates hypothesis-driven key capabilities in standing session in accordance with the present disclosure.
**FIG. 7** illustrates a comparative analysis of machine learning models for fall risk assessment in accordance with the present disclosure.
**FIG. 8A-B** is an exemplary flow chart illustrating a method for assessing fall risk in elderly individuals using a video game-based fall risk assessment system in accordance with the present disclosure.
**FIG. 9** illustrates a general computer architecture that can be appropriately configured to implement components disclosed in accordance with various embodiments of the present disclosure.

### DETAILED DESCRIPTION OF THE DISCLOSURE

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The present disclosure provides a technical solution that overcomes the problems encountered in prior art and offers a more accurate and reliable method for assessing fall risk, particularly for elderly individuals with chronic conditions such as diabetes. By leveraging machine learning algorithms and analyzing gameplay performance parameters, the invention enables a nuanced understanding of balance, postural stability, and motor control. This method addresses the limitations of traditional fall risk assessment tools, which often lack sensitivity and fail to account for specific vulnerabilities associated with conditions like peripheral neuropathy and cognitive decline. Ultimately, this innovative approach aims to enhance predictive accuracy and support early intervention strategies to reduce the incidence of falls and improve the overall quality of life for at-risk populations.

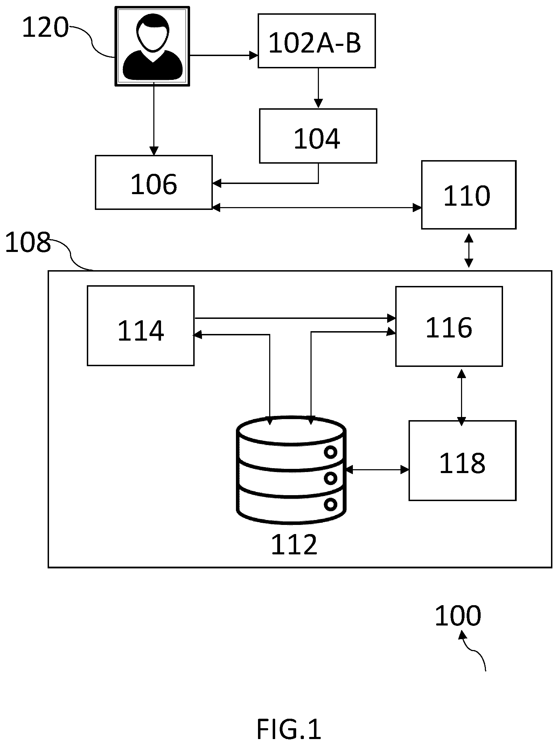

Now referring to **FIG. 1, FIG. 1** illustrates video game-based fall risk assessment system in accordance with the present disclosure. The system 100 includes a pair of sensor-equipped insoles 102A-B, an electronic control unit 104, a smart device 106, a server 108 and a communication network 110.

The pair of sensor-equipped insoles 102A-B embedded within a pair of size-matched gaming slippers. The pair of sensor-equipped insoles 102A-B serve as control units for steering of video games with eight pressure sensors positioned at defined positions within the heel, lateral arch, metatarsals 1, 3, and 5, hallux, and toes. These sensors quantify plantar pressures with a sensitivity of 3.4 mbar, ranging from 250 mbar to 7 bar, and a maximum sampling rate of 500 Hz. The electronic control unit 104 is configured to receive and process data from the pressure sensors, detect foot side automatically, and store data with internal memory. The electronic control unit 104 may include 16 GB internal storage, and a 10-hour energy supply. The pair of sensor-equipped insoles 102A-B may be covered with a protective sponge material and integrated into the gaming slipper with a supportive ethylene-vinyl acetate (EVA-50) layer.

During a gaming session, the individuals position their feet within the pairs of slippers and covering the insole. The games are played by changing the plantar pressure levels without movement of the feet. The pair of sensor-equipped insoles 102A-B is initialized to measure plantar foot pressures at 200 Hz and transmit the data to the video game application presented on the smart device 106 via Bluetooth 5.0 in real-time. The system facilitates the individual 120 to play video games both in a seated and standing position by modulating plantar foot pressure values. Each gaming session included four games. The four games include Apple-Catch (AC) where the individual 120 operate a carriage in an autumnal scenario using the plantar pressure to catch apples falling from a tree. Precision in maintaining target pressure is required for optimal carriage positioning. After each attempt, the carriage automatically returns to a central baseline. The four games include Balloon-Flying (BF) where the individual 120 maneuver a balloon across a skyline, with altitude controlled by varying forefoot pressure. The individual 120 avoids obstacles and collect points represented by smileys, requiring quick adjustments to avoid collisions and restarts. The four games include Cross-Pressure (CP) where the individual 120 apply pressure as indicated by green (low) and yellow (high) markers on specific foot areas for set durations. Achievements are confirmed through graphical indicators, and the game advances if no valid response is recorded within 25 seconds. The four games include Island-Jump (IJ) where the individual 120 guides a virtual bird across islands using plantar pressure to control jump distances and directions. Failure to adjust pressure within the indicated range results in the bird falling and the need to repeat the attempt. Optimal scores require adherence to predefined pressure thresholds, with deviations prompting restarts.

In an example embodiment, each individual 120 is subjected to play four consecutive games, initially in a seated position, followed by an optional repetition in standing position. Headphones may be provided to enhance patient attention to the game. Prior to the seated gaming session, the individual 120 underwent a calibration process for the pair of sensor-equipped insoles that involved standardized steps aimed at normalizing pressure measurements to a range of 0 to 1. The standardization steps are optional, however enhance the accuracy of prediction. As first step the individual 120 was seated in a balanced position and apply minimal and maximal pressures to the insoles for five seconds each. Furthermore, the individual 120 was instructed to stand up for five seconds and then maintain balance on both or single feet for an additional five seconds. Areas of pressure application were again indicated by yellow/orange/red color. The entire sensor calibration process lasted about two minutes. Thereafter, the individual 120 listened to standardized tutorials and engaged in interactive practice of pressure application to familiarize themselves with the game controls and setup. Each gaming session lasted about 10 minutes. Safety was a priority during the game-based assessments, and all sessions were conducted under the supervision of trained personnel. The individual 120 was closely monitored to ensure that they maintained their balance throughout the standing session. The server 108 includes a plurality of modules stored in a database 112. The plurality of modules of the server 108 includes a data receiving module 114 configured to receive gameplay data from the smart device. The plurality of modules of the server 108 includes a machine learning model 116 configured to extract specific game performance the during gameplay. Specific parameters were delineated for each game task and utilized to evaluate game performance. The specific game parameters include reaction time, anticipation time, pressure application accuracy, time within target zone, time outside target zone, and success rate.

The game performance parameters for the Apple-Catch (AC) Game includes the following: Reaction Time (s): This measures the time elapsed from the moment the apple starts falling until the participant initiates movement to adjust the carriage. A shorter reaction time indicates faster sensorimotor coordination, crucial for timely adjustments in response to environmental stimuli. Anticipation Time (s): This metric captures the time the participant spends preparing for the apple's descent before making any carriage movement. Prolonged anticipation time may reflect hesitancy or over-cautiousness, possibly indicative of reduced confidence in motor control or cognitive slowing. Time inside the Catching Area (s): The duration for which the apple remains within the designated target area, indicating the participant's ability to align the carriage with the apple's path. Longer times suggest improved balance and precise motor control. Time Outside the Catching Area (s): This represents the time the apple spends outside the optimal catching zone. Increased time outside the area may indicate difficulty in maneuvering the carriage, potentially signaling impaired coordination. Frequency Outside the Catching Area (n): The number of times the apple exits the target area as it falls. Higher frequencies reflect greater instability or challenges in maintaining carriage alignment, which may be linked to motor impairment. Final Virtual Distance (d): The distance between the final apple position and the center of the target area. Greater distances indicate poorer precision in positioning the carriage, highlighting deficiencies in motor accuracy or control. Apple Caught (Yes/No): A binary indicator of task success, assessing whether the apple was successfully caught by the carriage. This feature provides a direct measure of task performance and motor coordination. Normalized Pressure: The pressure applied by the participant's feet on the sensors during task performance, normalized over time. Maintaining appropriate pressure is essential for accurate carriage movement, with deviations potentially reflecting imbalances or incorrect weight distribution. Pressure Differences Between Successive Frames: This captures the change in pressure between consecutive time frames. Large fluctuations could indicate instability in the participant's balance or uneven weight shifts during the task. Pressure Gradients Between Successive Frames: This feature examines the rate of pressure change across successive frames. A smoother gradient reflects more controlled, stable pressure application, whereas abrupt changes may suggest poor balance or motor control. Pressure Time Integrals: This metric represents the cumulative pressure applied over the course of the task. It reflects the overall force exerted, which can provide insights into how participants distribute weight over time to control the carriage.

The game performance parameters for the Balloon-Flying (BF) Game includes the following: Smiley Count (n): This feature captures the number of smiley faces collected by the participant during the task. Smiley collection reflects the player's ability to navigate accurately and effectively, with higher counts indicating better coordination and task performance. Collision Frequency (n): The number of times the balloon collides with obstacles during the flight. A higher collision frequency suggests difficulty in controlling the balloon's altitude, potentially indicating reduced motor control or slower reaction times. Minimal Virtual Distance to Smiley 1-4: These parameters measure the closest distance between the balloon and each smiley during the task. A smaller distance indicates better precision in maneuvering the balloon to collect smileys without excessive deviations. Virtual Deviation from Ideal Flying Route: This parameter quantifies how much the balloon deviates from the optimal flight path. A larger deviation indicates greater difficulty in maintaining the desired trajectory, potentially signaling balance or coordination issues. Normalized Pressure: The level of pressure applied by the participant's forefoot to control the balloon's altitude. Maintaining appropriate pressure is crucial for smooth altitude adjustments. Pressure Differences Between Successive Frames: This feature measures how the pressure changes between consecutive time frames. Greater pressure variability may indicate instability in control or difficulty maintaining steady pressure. Pressure Gradients Between Successive Frames: The rate of change in pressure across time. A smoother gradient indicates better control, while abrupt changes suggest a lack of coordination. Pressure Time Integrals: This metric captures the total pressure applied over the duration of the task, reflecting the participant's effort and consistency in pressure application.

The game performance parameters for the Cross-Pressure (CP) Game includes the following: Anticipation Time (s): The time elapsed between the initiation of the task and the participant's first pressure application. Shorter anticipation times indicate faster response and reaction abilities, while longer times may suggest delays in motor coordination or hesitation. Time Outside Optimal Pressure Zone (s): This metric measures the amount of time the participant spends applying pressure that deviates from the target zone (either too high or too low). Longer durations outside the optimal zone may indicate difficulty in maintaining the required pressure, reflecting poor motor control. Relaxation Time (s): The time required for the participant to release pressure after applying the target force. A shorter relaxation time indicates better control over the release phase, whereas longer times may suggest slow motor function. Normalized Pressure (left or right foot): The actual pressure applied by each foot, normalized over time. This parameter evaluates how accurately the participant can maintain the required pressure for a given task. The normalized pressure provides insights into overall motor performance and precision. Pressure Differences Between Successive Frames (left or right foot): This feature captures the variation in pressure between consecutive time frames during the task. Large pressure differences may indicate inconsistent control or an inability to smoothly transition between pressure levels. Pressure Gradients Between Successive Frames (left or right foot): The rate of change in pressure over time, indicating the smoothness or abruptness of the pressure application. A smooth gradient suggests controlled and steady pressure application, while sharp gradients may reveal instability or difficulty in maintaining target pressure. Pressure Time Integrals (left or right foot): The cumulative pressure applied over the entire duration of the task. This metric reflects the participant's effort in applying and maintaining pressure, and it helps assess their ability to consistently meet the required target over time.

The game performance parameters for the Island-Jump (IJ) Game includes the following: Attempt Count (n): The number of attempts made to successfully guide the bird from one island to another. Multiple attempts may indicate difficulty in controlling pressure or reacting to the task demands. Deviation from Optimal Pressure (%): This feature captures how far the applied pressure deviates from the predefined optimal range required to successfully complete the jump. A greater deviation suggests that the participant is struggling to maintain the required force, leading to potential restarts. Anticipation Time (s): The time between task initialization and the participant's first pressure application. Shorter anticipation times reflect faster cognitive-motor responses, while longer times may indicate hesitation or slower reaction times. Execution Time (s): This is the time taken by the participant to apply the required pressure to guide the bird across the islands. Efficient execution is essential to avoid deviations that cause the bird to fall. Mean Pressure During Execution Phase: This parameter captures the average pressure applied during the execution phase of the jump. Maintaining pressure close to the predefined target is critical for successfully completing the task. Pressure Differences Between Successive Frames: The variation in pressure applied between consecutive frames. A higher difference indicates instability or inconsistency in maintaining the target pressure during the task. Pressure Gradients Between Successive Frames: The rate of pressure change over time. A smooth gradient suggests controlled and gradual pressure adjustment, while abrupt changes may reflect difficulty in maintaining steady control. Pressure Time Integrals: The cumulative pressure applied over the entire task duration. This metric provides insights into how consistently the participant applies pressure throughout the task, which is essential for understanding their ability to maintain motor control.

The machine learning model 116 analyzes the extracted game performance parameters to assess balance, postural stability, and motor control performance of the individual 120. The machine learning model 116 classifies the individual 120 based on the analysis of the extracted game performance parameters into the categories *"*no/low risk of falls" or "high risk of falls", wherein the classification considers posture-specific risks associated with both seated and standing positions by selecting and ranking the most predictive features for fall risk assessment. The machine learning model is able to classify with data from the seated game performance alone. The machine learning model 116 is trained using on both seated and standing gameplay data to evaluate fall risk, utilizing predefined hypotheses and statistical analysis techniques, which include feature selection processes and cross-validation methods. The training module 118 enhances the machine learning model's 116 accuracy in assessing fall risk based on gameplay data received from the smart device 106. Initially, the module extracts specific game performance parameters, including reaction time, anticipation time, pressure application accuracy, time within the target zone, time outside the target zone, and success rate. This data undergoes rigorous analysis to evaluate the individual's balance, postural stability, and motor control performance, utilizing both seated and standing gameplay data. To refine the analysis, predefined hypotheses and statistical techniques are employed, incorporating feature selection processes to identify the most relevant parameters and cross-validation methods to ensure the model's generalizability. Ultimately, the model classifies individuals into categories of "no/low risk of falls" or "high risk of falls", considering posture-specific risks. This iterative training process not only improves the model's predictive capabilities but also addresses the urgent need for effective fall risk assessment tools, particularly for vulnerable populations.

In an embodiment, the game performance parameters are grouped into task combinations (TCs) based on game tasks, with secondary features being calculated, including sum, mean, and standard deviation of primary features across the tasks. Taking apple-catching game as an example, TCL1 encompassed all tasks requiring the left foot to control carriage movement for apple collection, while TCR1 comprised that relied on the right foot. The sum, mean, and standard deviation of primary features across game tasks within TCs were classified as secondary features. For example, in the apple-catching game the reaction time for TCL1 was considered a secondary feature, calculated as the average reaction time across tasks involving the left foot. Overall, a total of 5,244 distinct features reflecting the individual's performance across the four games were extracted for each participant's gaming data set.

The statistical analysis provides robust support for the development of a fall risk prediction model based on game feature data. Descriptive statistics are meticulously reported for both categorical and continuous variables, with appropriate measures such as means and standard deviations for normally distributed data, and medians with interquartile ranges for non-normally distributed data. The use of Chisquare tests, t-tests, and Mann-Whitney U tests for group comparisons ensures reliable differentiation between fall risk groups. Advanced statistical methods, including the Shapiro-Wilk test for normality and adjustments like Yates' continuity correction and Fisher's exact test, enhance the reliability of the findings. Machine learning algorithms such as gradient boosting machines (GBM), elastic-net regularized generalized linear models (GLMNET), and random forests were employed to handle the high-dimensional and imbalanced dataset, which contained more predictors than observations. The inclusion of elastic-net regularization, focusing on both variable selection and parameter shrinking, along with GBM's sequential learning approach, further strengthens the model's ability to identify subtle patterns related to fall risks. Rigorous feature selection methods were used to eliminate multicollinearity, and cross-validation procedures were employed to ensure model generalizability and prevent overfitting. By utilizing Cohen's Kappa as a performance metric and optimizing the cut-off threshold with Youden's Index, the study achieves high accuracy in classifying individuals 120 with or without a risk of falls. This comprehensive statistical and machine learning framework justifies the claim that the game-based features can reliably predict fall risk in elderly individuals 120.

The system 100 assesses fall risk in individuals 120 by analyzing key motor and cognitive capabilities through hypothesis-driven analyses. The platform was designed to evaluate six essential pressure application capabilities that contribute to locomotion and fall prevention: Reaction Time: Assesses the speed and accuracy of task comprehension, crucial for reacting to sudden situations; Sensation: Measures precision in applying pressure during tasks, reflecting motor control necessary for stability; Skillfulness: Aggregates performance across tasks, indicating coordination, precision, and adaptability; Muscle Strength: Evaluates the ability to maintain force for body stabilization; Balance: Tracks plantar pressure distribution for postural control, essential for fall prevention; Endurance: Assesses sustained motor output over time, important for maintaining stability during extended tasks.

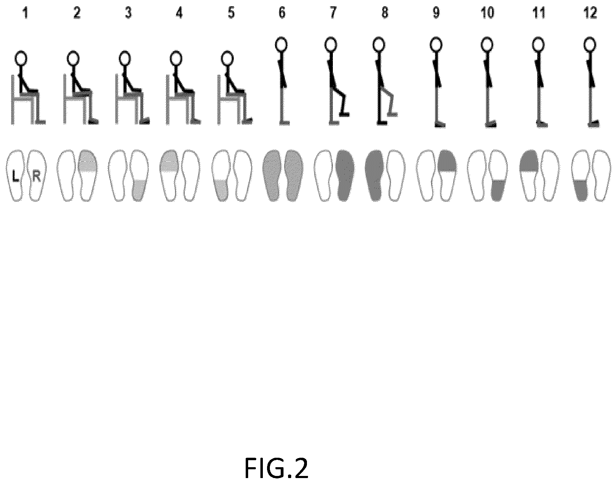

Now referring to **FIGS. 2, FIG. 2** illustrates the insole calibration process in accordance with the present disclosure. **FIG. 2** presents the visualization of the 12-step calibration procedure where the individuals apply minimal and maximal pressure at designated foot regions (forefoot/heel, left/right) in both seated and standing positions. These tasks ensure the proper calibration of the sensor-equipped insoles by guiding the individual through actions such as applying maximum pressure on different parts of the feet and maintaining balanced sitting and standing postures. The calibration process aims to normalize pressure readings across these specific foot regions to ensure accurate and consistent measurements during gameplay for fall risk assessment. The 12 steps include the following. 1 - Balanced sitting (5 sec): The individual sits in a balanced position with even pressure distribution across both feet for 5 seconds; 2 - Maximum pressure on the right forefoot (5 sec): The individual applies maximum pressure on the right forefoot while seated for 5 seconds; 3 - Maximum pressure on the right heel (5 sec): The individual applies maximum pressure on the right heel while seated for 5 seconds; 4 - Maximum pressure on the left forefoot (5 sec): The individual applies maximum pressure on the left forefoot while seated for 5 seconds; 5 - Maximum pressure on the left heel (5 sec): The individual applies maximum pressure on the left heel while seated for 5 seconds; 6 - Balance standing (5 sec): The individual stands in a balanced position, distributing weight evenly across both feet for 5 seconds; 7 - Standing on the right foot (5 sec): The individual stands solely on the right foot, maintaining balance for 5 seconds; 8 - Standing on the left foot (5 sec): The individual stands solely on the left foot, maintaining balance for 5 seconds; 9 - Maximum pressure on the right forefoot while standing (5 sec): The individual applies maximum pressure on the right forefoot while standing for 5 seconds; 10 - Maximum pressure on the right heel while standing (5 sec): The individual applies maximum pressure on the right heel while standing for 5 seconds; 11 - Maximum pressure on the left forefoot while standing (5 sec): The individual applies maximum pressure on the left forefoot while standing for 5 seconds; 12 - Maximum pressure on the left heel while standing (5 sec): The individual applies maximum pressure on the left heel while standing for 5 seconds. Each step ensures that pressure is applied systematically to different foot regions, ensuring consistent calibration of the insole sensors for accurate data collection during gameplay or other applications. The minmax plantar pressure normalization algorithm may be employed to adjust pressure measurements within a standardized range (0 to 1) for consistent data collection across participants, enhancing the accuracy of game performance analysis. In a setting without calibration steps, the sensor pressure detection range may be adjusted to age, gender, weight, height, and body-mass-index (BMI).

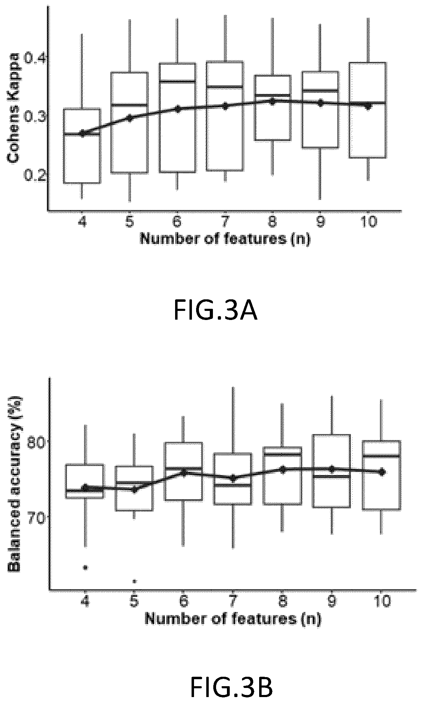

Now referring to **FIGS. 3A-B, FIGS. 3A-B,** illustrates the performance of a gradient boosting machine (GBM) model used to assess the fall risk by analyzing various extracted game performance parameters in accordance with the present disclosure. The **FIGS. 3A-B,** demonstrates how the model's performance, measured by Cohen's Kappa (FIG. 3A) and balanced accuracy (FIG. 3B), varies depending on the number of features (game performance parameters) used, ranging from 4 to 10 features. The boxplots show the distribution of performance across multiple iterations, with the black line indicating the mean values which is relevant to the system's ability to classify fall risk ("no/low" or "high") based on key game metrics such as reaction time, pressure application accuracy, and time in target zones, as mentioned in the claims. The optimal number of features ensures improved balance and motor control predictions for fall risk assessment.

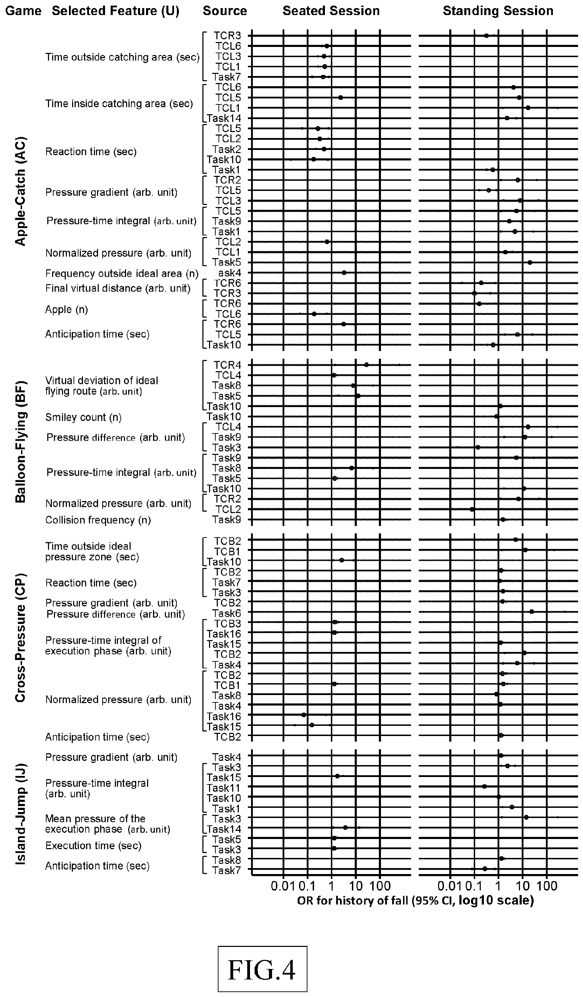

Now referring to **FIG. 4, FIG.4** illustrates a forest plot of game-based fall risk factors in accordance with the present disclosure. The forest plot illustrating the odds ratios (OR) for selected game performance features across four different video games (Apple-Catch, Balloon-Flying, Cross-Pressure, and Island-Jump) that correlate with the risk of falls in both seated and standing sessions. The x-axis represents the odds ratio on a logarithmic scale, with the dashed vertical line indicating an OR of 1 (no effect). The y-axis lists the selected game features, such as reaction time, time inside/outside the catching area, pressure gradients, and normalized pressure for each game, as well as task combinations for the left foot (TCL) and right foot (TCR), grouping specific foot pressure tasks. Each feature is evaluated for its correlation with fall risk, with confidence intervals (Cl) for the OR shown as horizontal lines. The odds ratio (OR) was calculated for each feature, with certain metrics such as time outside the catching area and pressure gradients in the Apple-Catch game, smiley count and deviation in the Balloon-Flying game, and anticipation time in the Cross-Pressure game, among others, being analyzed for their relationship with fall risk. Higher OR values indicate a stronger association with a risk of falls. This analysis is presented for both seated and standing positions, reflecting how fall risk factors may vary depending on posture during gameplay. The plot demonstrates the predictive value of game-derived metrics for assessing an individual's fall risk. Each game feature is a key indicator for analyzing balance, motor control, and postural stability, which contribute to determining the fall risk category of an individual based on the gameplay data.

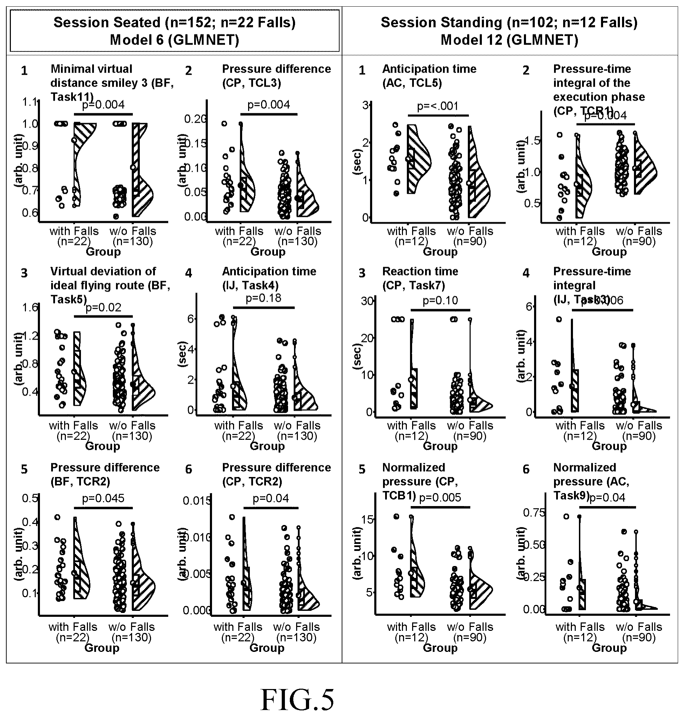

Now referring to **FIG. 5, FIG. 5** illustrates gaming performance metrics as predictors of fall risk in seated and standing conditions factors in accordance with the present disclosure. **FIG. 5** illustrates a comparison between seated and standing gaming sessions for elderly individuals with no/low or high risk of falls, highlighting specific gameplay metrics that correlate with fall risk. For each model, six features are visualized using rain cloud plots. The seated session uses GLMNET (lasso and elastic-net regularized generalized linear models), while the standing session uses GBM (gradient boosting machine). Data is presented from various games, including the Apple-Catch (AC), Balloon-Flying (BF), Cross-Pressure (CP), and Island-Jump (IJ) games. Additionally, task combinations are categorized as TCL (left foot) and TCR (right foot). In the seated session (GLMNET model), key features such as minimal virtual distance in the Balloon-Flying game, pressure differences in both feet during the Cross-Pressure game, and virtual deviation from ideal flying route show significant correlations with a risk of falls. In contrast, the standing session (GBM model) emphasizes the importance of normalized pressure, pressure-time integrals, and reaction time as strong predictors of fall risk, with reaction time demonstrating the highest statistical significance (p < 0.001). These findings suggest that gameplay metrics in both seated and standing tasks can provide critical insights into an individual's fall risk, enabling targeted interventions for fall prevention.

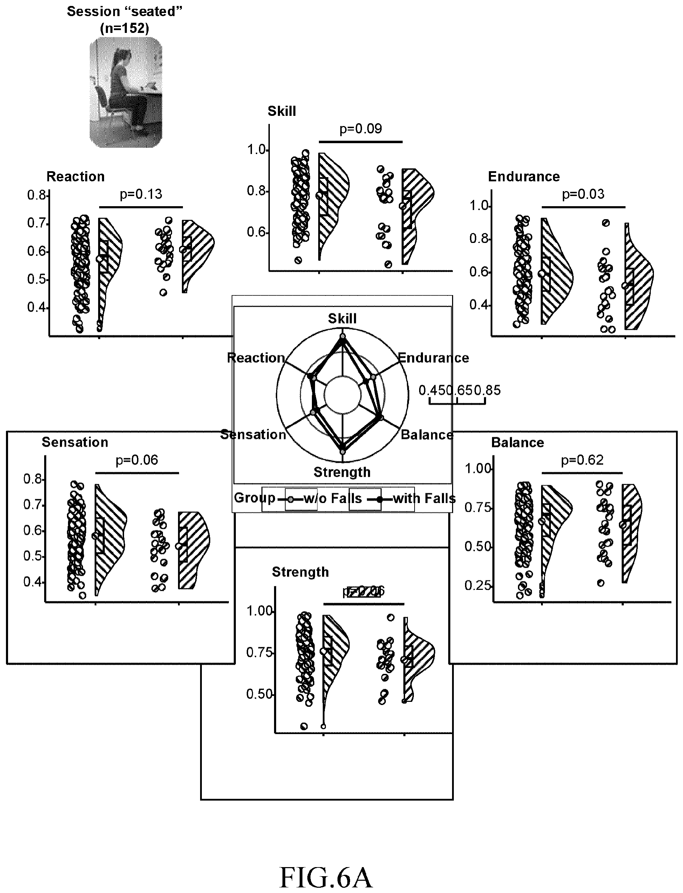

Now referring to **FIG. 6A**, **FIG. 6A** illustrates hypothesis-driven key capabilities in seated session in accordance with the present disclosure. **FIG. 6A** presents the six critical game performance parameters in elderly individuals performing a seated session (n=152). These parameters-skillfulness, endurance, balance, reaction, sensation, and muscle strength-are visualized through rain cloud plots and a spider diagram. The data distinguishes participants with falls (n=22, red) from those without falls (n=130, blue). Each parameter shows varying degrees of separation between the two groups. For example, endurance shows a statistically significant difference (p=0.03) between groups, while others, like balance and muscle strength, show nonsignificant differences (p=0.62, p=0.06). The central spider diagram provides an overall view of the performance metrics, visually highlighting the variance in physical capabilities between individuals who experienced falls and those who did not.

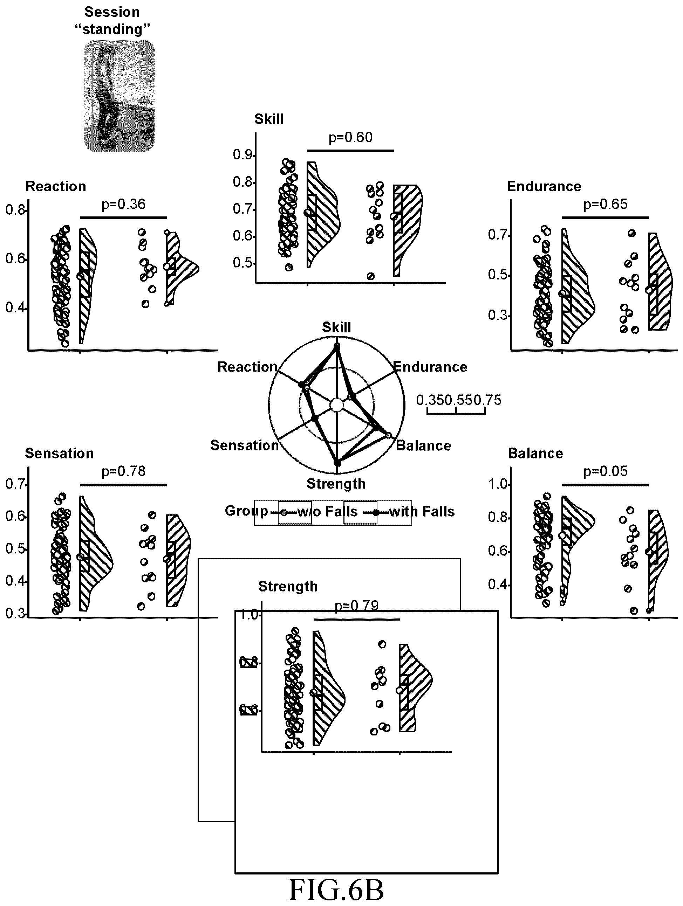

Now referring to **FIG. 6B**, **FIG. 6B** illustrates hypothesis-driven key capabilities in standing session in accordance with the present disclosure. **FIG. 6B** presents the six critical game performance parameters in elderly individuals performing a standing session (n=102). These parameters (i.e.) skillfulness, endurance, balance, reaction, sensation, and muscle strength are visualized through rain cloud plots and a spider diagram. The data distinguishes participants with falls (n=12, red) from those without falls (n=90, blue). Each parameter shows varying degrees of separation between the two groups. For example, balance shows a statistically significant difference (p=0.047) between groups, while others, like endurance and muscle strength, show nonsignificant differences (p=0.65, p=0.79). The central spider diagram provides an overall view of the performance metrics, visually highlighting the variance in physical capabilities between individuals who experienced falls and those who did not.

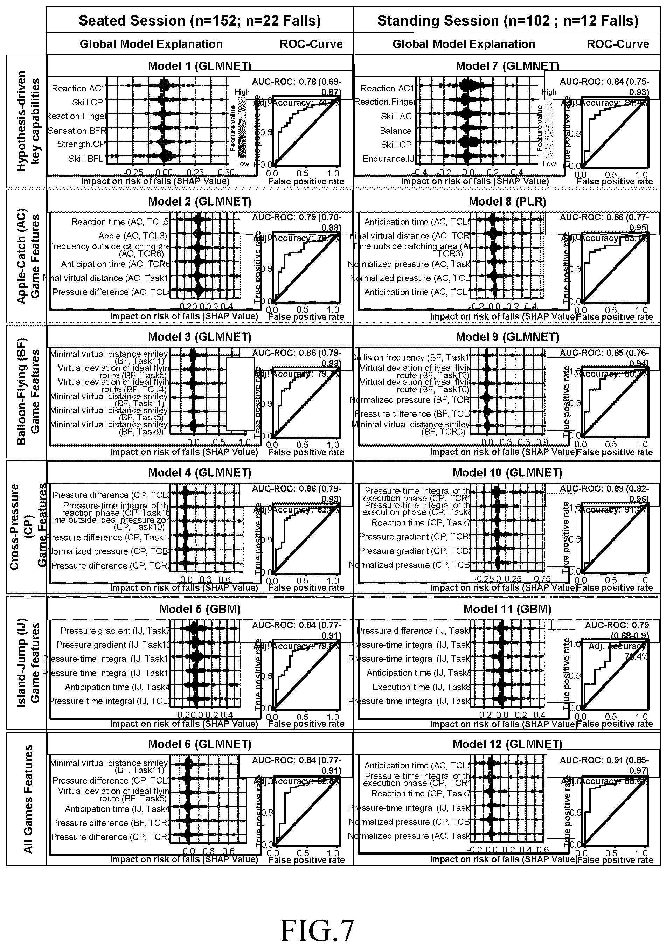

Now referring to **FIG. 7, FIG. 7** illustrates a comparative analysis of machine learning models for fall risk assessment in accordance with the present disclosure. Models 1-6 were trained using 152 data sets from the "seated" gaming session, while 102 data set from the "standing" gaming session were used for models 7-12. The sources of model features are listed by importance, with final features (n=6) ranked using the "varlmp" function from the R Caret library. A SHAP summary dot plot ranks these features, where the probability of falls increases with the SHAP value of a feature. Each participant is represented by a dot, with bright yellow indicating higher feature values and dark purple indicating lower values. SHAP values above zero indicate a tendency towards the "high risk of falls" class. A ROC curve further illustrates the predictive performance of each model, with adjusted accuracy calculated using the optimal predicted probability cutoff as determined by Youden's Index. The machine learning approaches include methods such as GLMNET (lasso and elastic-net regularized generalized linear models), GBM (gradient boosting machine), and PLR (penalized logistic regression), utilizing data from specific games such as Apple-Catch (AC), Balloon-Flying (BF), Cross-Pressure (CP), Island-Jump (IJ), and task combinations for both the left (TCL) and right (TCR) feet.

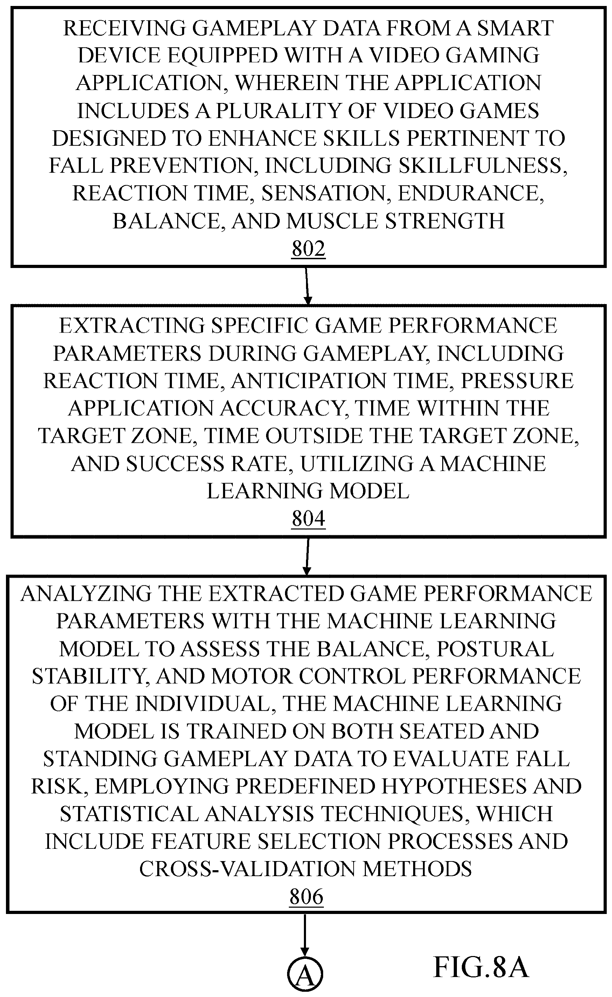

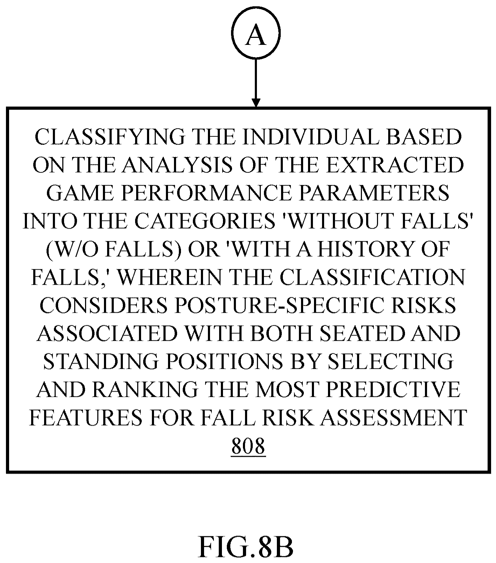

With reference to **FIGS. 1-7****,** **FIG. 8A-B** is an exemplary flow chart illustrating a method for assessing fall risk in elderly individuals using a video game-based fall risk assessment system in accordance with the present disclosure. At step 802, the method performs a step of receiving gameplay data from a smart device equipped with a video gaming application, wherein the application includes a plurality of video games designed to enhance skills pertinent to fall prevention, including skillfulness, reaction time, sensation, endurance, balance, and muscle strength. At step 804, the method performs a step of extracting specific game performance parameters during gameplay, including reaction time, anticipation time, pressure application accuracy, time within the target zone, time outside the target zone, and success rate, utilizing a machine learning model. At step 806, the method performs a step of analyzing the extracted game performance parameters with the machine learning model to assess the balance, postural stability, and motor control performance of the individual, wherein the machine learning model is trained on both seated and standing gameplay data to evaluate fall risk, employing predefined hypotheses and statistical analysis techniques, which include feature selection processes and cross-validation methods. At step 806, the method performs a step of classifying the individual based on the analysis of the extracted game performance parameters into the categories "no/low risk of falls" or "high risk of falls", wherein the classification considers posture-specific risks associated with both seated and standing positions by selecting and ranking the most predictive features for fall risk assessment. To avoid repetition, each component of the video game-based fall risk assessment system 100 and its function are not explained herein, which are explained through **FIGS 1-7****.**

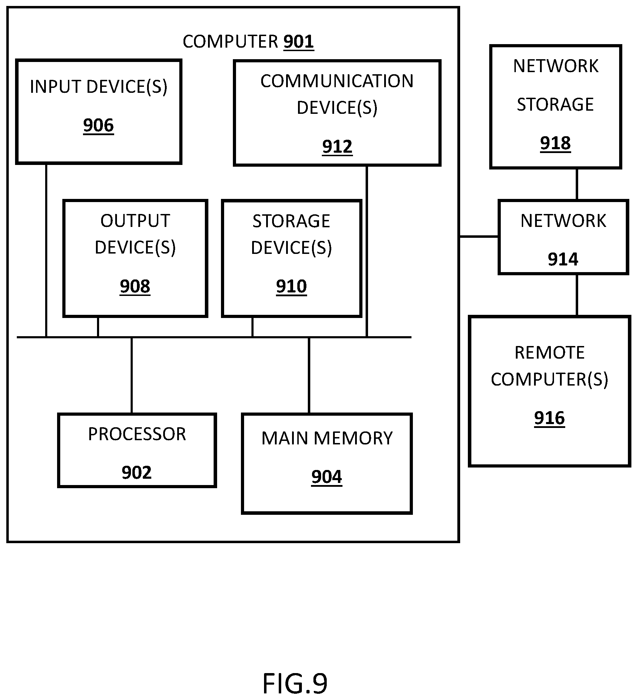

With reference to **FIG. 9, FIG. 9** illustrates a general computer architecture that can be appropriately configured to implement components disclosed in accordance with various embodiments of the present disclosure. The general computing architecture 900 can include various common computing elements, such as a computer 901, a network 914, and one or more remote computers 916. The computer 901 may be a server, a desktop computer, a laptop computer, a tablet computer or a mobile computing device. The computer 901 may include a processor 902, a main memory 904 and a system bus. The processor 902 may feature one or more processing units that can operate independently of each other. The main memory 904 may include volatile devices, non-volatile devices, or other random access memory devices. The computer 901 may feature secondary storage 910, consisting of one or more removable and/or non-removable storage units. These units house an operating system that manages various applications on the computer 901. The secondary storage 910 may also be used to store software configured to implement the components of the embodiments disclosed herein, which may be executed as one or more applications under the operating system. The computer 901 may also include a communication device(s) 912 through which the computer communicates with other devices, such as one or more remote computers 916, over wired and/or wireless computer networks 914. The communication device(s) 912 may communicate over but not limited to Wi-Fi, Bluetooth, ultra-wide band technology, and mobile telephone networks. The computer 901 may also access network storage 918 through computer network 914. The network storage 918 may include a networkattached storage device or cloud-based storage. The operating system and/or software may be stored in network storage 918. The computer 901 may have various input device(s) 906 for example, keyboard, mouse, touchscreen, camera, microphone, or a sensor, output device(s) 908, for example, a display, speakers or a printer. Storage devices 910, the communication device(s) 912, input devices 906 and output devices 908 may be integrated within a computer system or connected through various computer input/output interface devices.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the appended claims.

### LIST OF REFERENCE NUMERALS

100 - video game-based fall risk assessment system
102A-B - sensor-equipped insoles
104 - electronic control unit
106 - smart device
108 - server
110 - communication network
112 - database
114 - data receiving module
116 - machine learning model
118 - training module 118
120 - individual

With the above context, the present invention also relates to the following consecutively numbered embodiments:
1. A video game-based fall risk assessment system (100) for assessing fall risk in an elderly individual (120), comprising:
   a pair of sensor-equipped insoles (102A-B) embedded within a pair of size-matched gaming slippers, **characterized in that** each insole (102A-B) comprise a number of pressure sensors positioned at defined locations below a foot of the individual (120), the foot including heel, lateral arch, metatarsals 1, 3, and 5, hallux, and toes;
   an electronic control unit (104) configured to receive and process plantar foot pressure data from the pressure sensors, detect foot side automatically, and store data with an internal memory;
   a video gaming application installed on a smart device (106) configured to receive the plantar foot pressure data from the electronic control unit (104) in real-time, wherein the gaming application comprising four video games, wherein each video game is designed to address capabilities pertinent to prevent fall comprising skillfulness, reaction time, sensation, endurance, balance, and muscle strength, wherein the individual interacts with the games by modulating the plantar foot pressures applied through the pair of sensor-equipped insoles (102A-B) to control throughout virtual tasks within the games;
   a server (108); and
   a memory communicably connected to the server (108) and storing instructions that, when executed by the server (108), cause the server (108) to:
      receives gameplay data from the smart device (106);
      extract specific game performance parameters during the gameplay, including reaction time, anticipation time, pressure application accuracy, time within target zone, time outside target zone, and success rate, using a machine learning model (116);
      analyze the extracted game performance parameters with the machine learning model (116) to assess the balance, postural stability, and motor control performance of the individual (120), wherein the machine learning model (116) is trained on both seated and standing gameplay data to evaluate fall risk, utilizing predefined hypotheses and statistical analysis techniques, which include feature selection processes and cross-validation methods;
      classify the individual based on the analysis of the extracted game performance parameters into the categories "no/low risk of falls" or "high risk of falls", wherein the classification considers posture-specific risks associated with both seated and standing positions by selecting and ranking the most predictive features for fall risk assessment.
2. The system (100) according to embodiment 1, **characterized in that** the predefined hypotheses are based on studies that correlate specific motor and cognitive skills with fall risk and the statistical analysis techniques comprise regression analysis, hypothesis testing, and correlation assessments to determine relationships between the game performance parameters and the fall risk.
3. The system (100) according to embodiment 1 or 2, **characterized in that** the feature selection process employs algorithms to eliminate multicollinearity among the extracted game performance parameters and the cross-validation methods involve k-fold validation.
4. The system (100) according to any one of the embodiments 1 to 3, **characterized in that** each sensor has a sensitivity of 3.4 mbar, ranging from 250 mbar to 7 bars, and operates with a maximum sampling rate of 500 Hz.
5. The system (100) according to any one of the embodiments 1 to 4, **characterized in that** the sensor-equipped insoles (102A-B) are initialized to measure the plantar foot pressures at a frequency of 200 Hz and transmit the measured plantar foot pressure data to the video game application presented on the smart device (106) in real-time.
6. The system (100) according to any one of the embodiments 1 to 5, **characterized in that** the four video games comprise,
   an apple-catching game requiring precision in maintaining target pressure for optimal positioning;
   a balloon-flying game controlled by forefoot pressure;
   a cross-pressure game requiring the application of specific pressures on designated foot areas; and
   an island-jumping game requiring the control of jump distances and directions using plantar pressures.
7. The system (100) according to any one of the embodiments 1 to 6, **characterized in that** the game performance parameters extracted from the apple-catching game include the reaction time, the anticipation time, time inside the catching area, time outside the catching area, frequency outside the catching area, final virtual distance, and apple-caught success.
8. The system (100) according to any one of the embodiments 1 to 7, **characterized in that** game performance parameters extracted from the balloon-flying game metrics include smiley count, collision frequency, minimal virtual distance to smiley, virtual deviation from ideal flying route, and normalized pressure.
9. The system (100) according to any one of the embodiments 1 to 8, **characterized in that** the game performance parameters extracted from the cross-pressure game metrics include the anticipation time, time outside the optimal pressure zone, relaxation time, and normalized pressure for each foot.
10. The system (100) according to any one of the embodiments 1 to 9, **characterized in that** the game performance parameters extracted from the island-jumping game metrics include attempt count, deviation from optimal pressure, the anticipation time, execution time, and mean pressure during the execution phase.
11. The system (100) according to any one of the embodiments 1 to 10, **characterized in that** the machine learning model (116) is configured to assess balance control of the individual (120) by analyzing pressure differences between successive frames and pressure gradients between successive frames.
12. The system (100) according to any one of the embodiments 1 to 11, **characterized in that** the game performance parameters are grouped into task combinations (TCs) based on game tasks, with secondary features being calculated, including sum, mean, and standard deviation of primary features across the tasks.
13. The system (100) according to any one of the embodiments 1 to 12, **characterized in that** the server (108) is configured to provide real-time feedback to the individual (120), including visual indicators of task success and graphical representations of applied pressure.
14. The system (100) according to claim 1, **characterized in that** the number of pressure sensors in each insole (102A-B) is between 6 and 12.
15. The system (100) according to claim 1, **characterized in that** the number of pressure sensors in each insole (102A-B) is 8.
16. A method for assessing fall risk in elderly individuals using a video game-based fall risk assessment system (100), **characterized in that** the method comprises:
   receiving gameplay data from a smart device (106) equipped with a video gaming application, wherein the application includes a plurality of video games designed to enhance skills pertinent to fall prevention, including skillfulness, reaction time, sensation, endurance, balance, and muscle strength;
   extracting specific game performance parameters during gameplay, including reaction time, anticipation time, pressure application accuracy, time within the target zone, time outside the target zone, and success rate, utilizing a machine learning model (116);
   analyzing the extracted game performance parameters with the machine learning model (116) to assess the balance, postural stability, and motor control performance of the individual (120), wherein the machine learning model (116) is trained on both seated and standing gameplay data to evaluate fall risk, employing predefined hypotheses and statistical analysis techniques, which include feature selection processes and cross-validation methods; and
   classifying the individual (120) based on the analysis of the extracted game performance parameters into the categories "no/low risk of falls" or "high risk of falls", wherein the classification considers posture-specific risks associated with both seated and standing positions by selecting and ranking the most predictive features for fall risk assessment.
17. The method according to embodiment 14, **characterized in that** the predefined hypotheses are based on studies that correlate specific motor and cognitive skills with fall risk and the statistical analysis techniques comprise regression analysis, hypothesis testing, and correlation assessments to determine relationships between game performance parameters and fall risk.
18. The method according to embodiment 14 or 15, **characterized in that** the feature selection process employs algorithms to eliminate multicollinearity among the extracted game performance parameters and the cross-validation methods involve k-fold validation.

## Claims

1. A video game-based fall risk assessment system (100) for assessing fall risk in an elderly individual (120), comprising:
a pair of sensor-equipped insoles (102A-B) embedded within a pair of size-matched gaming slippers, **characterized in that** each insole (102A-B) comprise a number of pressure sensors positioned at defined locations below a foot of the individual (120), the foot including heel, lateral arch, metatarsals 1, 3, and 5, hallux, and toes;
an electronic control unit (104) configured to receive and process plantar foot pressure data from the pressure sensors, detect foot side automatically, and store data with an internal memory;
a video gaming application installed on a smart device (106) configured to receive the plantar foot pressure data from the electronic control unit (104) in real-time, wherein the gaming application comprising four video games, wherein each video game is designed to address capabilities pertinent to prevent fall comprising skillfulness, reaction time, sensation, endurance, balance, and muscle strength, wherein the individual interacts with the games by modulating the plantar foot pressures applied through the pair of sensor-equipped insoles (102A-B) to control throughout virtual tasks within the games;
a server (108); and
a memory communicably connected to the server (108) and storing instructions that, when executed by the server (108), cause the server (108) to:
receives gameplay data from the smart device (106);
extract specific game performance parameters during the gameplay, including reaction time, anticipation time, pressure application accuracy, time within target zone, time outside target zone, and success rate, using a machine learning model (116);
analyze the extracted game performance parameters with the machine learning model (116) to assess the balance, postural stability, and motor control performance of the individual (120), wherein the machine learning model (116) is trained on both seated and standing gameplay data to evaluate fall risk, utilizing predefined hypotheses and statistical analysis techniques, which include feature selection processes and cross-validation methods;
classify the individual based on the analysis of the extracted game performance parameters into the categories "no/low risk of falls" or "high risk of falls", wherein the classification considers posture-specific risks associated with both seated and standing positions by selecting and ranking the most predictive features for fall risk assessment.

2. The system (100) according to claim 1, **characterized in that** the predefined hypotheses are based on studies that correlate specific motor and cognitive skills with fall risk and the statistical analysis techniques comprise regression analysis, hypothesis testing, and correlation assessments to determine relationships between the game performance parameters and the fall risk.

3. The system (100) according to claim 1, **characterized in that** the feature selection process employs algorithms to eliminate multicollinearity among the extracted game performance parameters and the cross-validation methods involve k-fold validation.

4. The system (100) according to claim 1, **characterized in that** each sensor has a sensitivity of 3.4 mbar, ranging from 250 mbar to 7 bars, and operates with a maximum sampling rate of 500 Hz.

5. The system (100) according to claim 1, **characterized in that** the sensor-equipped insoles (102A-B) are initialized to measure the plantar foot pressures at a frequency of 200 Hz and transmit the measured plantar foot pressure data to the video game application presented on the smart device (106) in real-time.

6. The system (100) according to claim 1, **characterized in that** the four video games comprise,
an apple-catching game requiring precision in maintaining target pressure for optimal positioning;
a balloon-flying game controlled by forefoot pressure;
a cross-pressure game requiring the application of specific pressures on designated foot areas; and
an island-jumping game requiring the control of jump distances and directions using plantar pressures.

7. The system (100) according to claim 6, **characterized in that** the game performance parameters extracted from the apple-catching game include the reaction time, the anticipation time, time inside the catching area, time outside the catching area, frequency outside the catching area, final virtual distance, and apple-caught success.

8. The system (100) according to claim 6, **characterized in that** game performance parameters extracted from the balloon-flying game metrics include smiley count, collision frequency, minimal virtual distance to smiley, virtual deviation from ideal flying route, and normalized pressure.

9. The system (100) according to claim 6, **characterized in that** the game performance parameters extracted from the cross-pressure game metrics include the anticipation time, time outside the optimal pressure zone, relaxation time, and normalized pressure for each foot.

10. The system (100) according to claim 4, **characterized in that** the game performance parameters extracted from the island-jumping game metrics include attempt count, deviation from optimal pressure, the anticipation time, execution time, and mean pressure during the execution phase.

11. The system (100) according to claim 1, **characterized in that** the machine learning model (116) is configured to assess balance control of the individual (120) by analyzing pressure differences between successive frames and pressure gradients between successive frames.

12. The system (100) according to claim 1, **characterized in that** the game performance parameters are grouped into task combinations (TCs) based on game tasks, with secondary features being calculated, including sum, mean, and standard deviation of primary features across the tasks.

13. The system (100) according to claim 1, **characterized in that** the server (108) is configured to provide real-time feedback to the individual (120), including visual indicators of task success and graphical representations of applied pressure.

14. The system (100) according to claim 1, **characterized in that** the number of pressure sensors in each insole (102A-B) is between 6 and 12.

15. The system (100) according to claim 1, **characterized in that** the number of pressure sensors in each insole (102A-B) is 8.

16. A method for assessing fall risk in elderly individuals using a video game-based fall risk assessment system (100), **characterized in that** the method comprises:
receiving gameplay data from a smart device (106) equipped with a video gaming application, wherein the application includes a plurality of video games designed to enhance skills pertinent to fall prevention, including skillfulness, reaction time, sensation, endurance, balance, and muscle strength;
extracting specific game performance parameters during gameplay, including reaction time, anticipation time, pressure application accuracy, time within the target zone, time outside the target zone, and success rate, utilizing a machine learning model (116);
analyzing the extracted game performance parameters with the machine learning model (116) to assess the balance, postural stability, and motor control performance of the individual (120), wherein the machine learning model (116) is trained on both seated and standing gameplay data to evaluate fall risk, employing predefined hypotheses and statistical analysis techniques, which include feature selection processes and cross-validation methods; and
classifying the individual (120) based on the analysis of the extracted game performance parameters into the categories "no/low risk of falls" or "high risk of falls", wherein the classification considers posture-specific risks associated with both seated and standing positions by selecting and ranking the most predictive features for fall risk assessment.

17. The method according to claim 14, **characterized in that** the predefined hypotheses are based on studies that correlate specific motor and cognitive skills with fall risk and the statistical analysis techniques comprise regression analysis, hypothesis testing, and correlation assessments to determine relationships between game performance parameters and fall risk.

18. The method according to claim 14, **characterized in that** the feature selection process employs algorithms to eliminate multicollinearity among the extracted game performance parameters and the cross-validation methods involve k-fold validation.
